# EUROPEAN PATENT SPECIFICATION

(11) **EP 1 333 832 B1**
(45) Date of publication and mention of the grant of the patent: **28.11.2007**
(21) Application number: 01983555.2
(22) Date of filing: 16.10.2001
(51) Int. Cl.: A61K 31/495, A61P 25/18

(54) **NOVEL USES OF COMBINED 5-HT1A AGONISTS AND SEROTONIN REUPTAKE INHIBITORS**
NEUE VERWENDUNG VON SUBSTANZEN MIT KOMBINIERTEN 5-HT1A AGONISTISCHEN UND SEROTONIN REUPTAKE INHIBITOR-AKTIVITÄTEN
UTILISATIONS NOUVELLES D'AGONISTES 5-HT1A ET D'INHIBITEURS DE RECAPTAGE DE SEROTININE COMBINES

(30) Priority: 14.11.2000 EP 00124815
(43) Date of publication of application: 13.08.2003
(73) Proprietor: Merck Patent GmbH, 64293 Darmstadt (DE)
(72) Inventor: BARTOSZYK, Gerd, 64331 Weiterstadt (DE)
(86) International application number: PCT/EP2001/011952
(87) International publication number: WO 2002/040024

(56) References cited:
- EP-A- 0 648 767
- EP-A- 0 736 525
- WO-A-00/05225
- WO-A-98/11068
- DE-A- 4 333 254
- DE-A- 19 730 989
- US-A- 5 532 241

## Description

The present invention relates to the use of compounds being combined selective serotonin (5-HT) reuptake inhibitors (SSRIs) and 5-HT_{1A} receptor agonists for the manufacture of a medicament for use in veterinary medicine for the treatment or prophylaxis of disorders associated with behavioral stressors.

Particularly, the present invention relates to the use of combined selective serotonin (5-HT) reuptake inhibitors (SSRIs) and 5-HT_{1A} receptor agonists chosen from the group consisting of 1-[4-(5-cyanoindol-3-yl)butyl]-4-(2-carbamoyl-benzofuran-5-yl)-piperazine or a physiologically acceptable salt thereof or 3-{4-[4-(4-cyano-phenyl)-piperazin-1-yl]-butyl}-1H-indole-5-carbonitrile or a physiologically acceptable salt thereof, for the manufacture of a medicament for use in veterinary medicine for the treatment of self directed traumatic disorders associated with behavioral stressors and/or compulsive disorders associated with behavioral stressors.

1-[4-(5-cyanoindol-3-yl)butyl]-4-(2-carbamoyl-benzofuran-5-yl)-piperazine, physiologically acceptable salts thereof (US 5,532,241, column 7, lines 30 to 58) and a process (US 5,532,241, Example 4) by which it/they can be prepared are known from U.S. Patent US 5,532,241. The compound which is referred to herein is described in the patent as a combined selective serotonin (5-HT) reuptake inhibitor (SSRI) and 5-HT_{1A} receptor agonist. Therefore, the use of 1-[4-(5-cyanoindol-3-yl)butyl]-4-(2-carbamoyl-benzofuran-5-yl)-piperazine and its physiologically acceptable acid addition salts for the manufacture of a medicament for the treatment of depressive disorders, including the sub-type disorders major depressive disorder and dysthymic disorder, for the treatment of anxiety disorders, for the treatment of psychiatric disorders like psychoses, schizophrenia or schizoaffective disorder, for the treatment of cerebral infarct like stroke and cerebral ischemia, for the treatment of CNS disorders such as tension, for the therapy of side-effects in the treatment of hypertension (e.g. with α-methyldopa) and for the prophylaxis and therapy of cerebral disorders (e.g. migraine) is disclosed. Additionally, the use in endocrinology and gynecology is described, e.g. for the treatment of acromegaly, hypogonadism, secondary amenorrhea, premenstrual syndrome or undesired puerperal lactation.

3-{4-[4-(4-Cyano-phenyl)-piperazin-1-yl]-butyl}-1H-indole-5-carbonitrile, physiologically acceptable salts thereof (EP 0 736 525, page 3, lines 5, 26 and, page 8 lines 28 to page 9 lines 12) and a process (EP 0 736 525, Example 1) by which it/they can be prepared are known from EP 0 736 525. They show, in particular, actions on the central nervous system, especially 5-HT1A-agonistic and 5-HT-reuptake inhibiting actions. Therefore they are suitable for the treatment of disorders of the central nervous system such as states of tension, depressions and/or psychoses and of side effects in the treatment of hypertension. Additionally, the use in endocrinology and gynecology is described, e.g. for the treatment of acromegaly, hypogonadism, secondary amenorrhea, premenstrual syndrome or undesired puerperal lactation, and furthermore for the prophylaxis and therapy of cerebral disorders (e.g. migraine), in particular in geriatrics, similarly to certein ergot alkaloids and for the control of the sequelae of cerebral infarcts (apoplexia cerebri), such as stroke and cerebral ischaemias.

The invention had the object of providing novel uses for compounds being combined selective serotonin (5-HT) reuptake inhibitors (SSRIs) and 5-HT_{1A} receptor agonists, in particular of 1-[4-(5-cyanoindol-3-yl)butyl]-4-(2-carbamoyl-benzofuran-5-yl)-piperazine and its physiologically acceptable salts or 3-{4-[4-(4-cyano-phenyl)-piperazin-1-yl]-butyl}-1H-indole-5-carbonitrile or a physiologically acceptable salt thereof, especially in the field of veterinary medicine.

It has been found that combined selective serotonin (5-HT) reuptake inhibitors (SSRIs) and 5-HT_{1A} receptor agonists, in particular 1-[4-(5-cyanoindol-3-yl)butyl]-4-(2-carbamoyl-benzofuran-5-yl)-piperazine or a physiologically acceptable salt thereof or 3-{4-[4-(4-cyano-phenyl)-piperazin-1-yl]-butyl}-1H-indole-5-carbonitrile or a physiologically acceptable salt thereof, also have activity against disorders associated with behavioral stressors in veterinary medicine.

Disorders associated with behavioral stressors include self directed traumatic disorders associated with behavioral stressors (also known as self-injurious behavior) and compulsive disorders associated with behavioral stressors (e.g. C.C. Pinney, The illustrated veterinary guide for dogs, cats, birds and exotic pets. McGraw Hill, 1992; U.A. Luescher, in: N.H. Dodman and L. Shuster (eds.): Psychopharmacology of Animal Behavior, Blackwell Science Inc., Malden, 1998, pp. 203-221). Self directed traumatic disorders associated with behavioral stressors and compulsive disorders associated with behavioral stressors result particularly in stereotyped behaviors in animals, a common problem for pet owners, breeders, zoo keepers and veterinarians. Self directed traumatic disorders are characterized by self-injurious behaviors such as acral lick dermatitis (ALD), flank sucking, spinning and tail chaising, nail biting and checking of the rear end in dogs, psychogenic alopecia (hair pulling), tail attacking, pawing the face and hyperesthesia in cats or feather picking (trichotillomania) in birds.

Compulsive disorders are seen in other species, too, e.g. repetitive or ritualized pacing seen in zoo animals, cribbing, self-mutation or weaving in horses.

For example, intense animal keeping is characterized by high animal populations on restricted grounds. Animals kept under such conditions experience extreme stress and develop both traumatic and compulsive disorders, e.g. bar biting in pigs in meat-producing farms or feather picking in hens in egg-producing farms (e.g. M. Kiley-Worthinton, Behavioral problems of farm animals, Orial Press, Stockfields, 1977; R.J. Young et al., Appl. Anim. Behav. Sci., 1994, 39: 237-247).

Amongst these disorders, in veterinary practice especially the self directed traumatic disorders, i.e. acral lick dermatitis in dogs, psychogenic alopecia in cats, feather picking in caged birds, and self-mutilation, cribbing and weaving in horses, are very common and usually the reason why pet owners consult a veterinarian (e.g. R.R. Keiper, Anim. Behav., 1970, 18: 353-357; C. Davis, Vet. Clin. N Am. Sm. An. Pract., 1991, 21: 1281-1288; C.C. Pinney, The illustrated veterinary guide for dogs, cats, birds and exotic pets. McGraw Hill, 1992; D.J. Stein and N.H. Dodman, Comp. Psychiatr., 1994, 35: 275-285).

Acral lick dermatitis is a condition characterized by excessive paw licking and scratching in dogs. This results in the characteristic dermatitis; other sequelae include acute and chronic osteomyelitis. Acral lick dermatitis, also known as lick granuloma, creates areas of hair loss and the production of lesions, which may range in size from several centimeters to the entire surface of the limb, finally inflamed and ulcerated causing discomfort, pain, and in severe cases crippling. Osteomyelitis represents a suppurative infection of bone tissue with a progressive course with signs of bone destruction and formation of atrophic foci in the bone; in some cases sequestra and false joints are formed. The disorder is seen in certain breeds of large dog (particularly prone are Doberman Pinscher, German Shephard, Great Danes, Golden Retriever, Labrador Retriever, Irish Setter, and Weimaraner), and may be more common in particular families within breeds (e.g. L. Veith, Canine Pract., 1986, 14: 15-22; J.J. Van Nes, J. Am. Vet. Med. Assoc., 1986, 198: 157-160; S.D. White, J. Am. Vet. Med. Assoc., 1990, 202: 1073-1076; B.A. Goldberger and J.L. Rapoport, J. Anim. Hosp. Assoc., 1990, 27: 179-182; U.A. Luescher et al., Vet. Clin. N Am. Sm. Anim. Pract., 1991, 21: 401-413; K. Overall, J. Am. Vet. Assoc., 1994, 205: 1733-1741; U.A. Luescher, Vet. Intern., 1998, 10: 7-12).

Psychogenic alopecia is found in cats, where excessive depilation leads to bare patches (e.g. U.A. Luescher et al., Vet. Clin. N Am. Sm. Anim. Pract., 1991, 21: 401-413; J.W.S. Bradshaw et al., J. Appl. An. Behav. Sci., 1997, 52: 373-379; J. Dehasse, Appl. An. Behav., 1997, 52: 365-371; L.S. Sawyer et al., J. Am. Vet. Med. Assoc., 1999, 214: 71-74). Animal behaviorists have typically viewed the disorder as stress related. All strains are prone of psychogenic alopecia.

Feather picking in birds is seen in a range of avian species. Complications associated with this disorder include severe haemorrhage, infection, and hypothermia. It is also known that stress and confinement play a role in this behavior. Very prone are African greys and Timneh greys, budgerigars, rosellas, neophemas and other Australian parakeets, cockatoos, conures, electus parrots, lorikeets, lovebirds, and macaws (e.g. R.R. Keiper, Anim. Behav., 1970, 18: 353-357; D. Alderton, An essential reference for keeping more than 200 parrot family species, Salamander Books, 1992; G.A. Gallerstein, The complete bird owner's handbook, Macmillan, 1994; C. Davis, Vet. Clin. N Am. Sm. An. Pract., 1991, 21: 1281-1288; F. Iglauer and R. Rasim, J. Sm. An. Pract., 1993, 34: 564-566; P.S: Brodnick et al., J. Behav. Ther. Exp. Psychiatr., 1994, 25: 189-196; S. Blanchard, Pet Bird Report, 1995, 23; S.V. Juarbe-Diaz, J. Am. Vet. Med. Assoc., 2000,1562-1564).

Self-mutilation behavior in horses consists of self-biting (flanks, limbs, lateral thoracic wall, pectoral area, tail) and other uncontrollable violent behaviors typically including spinning in circles, bucking, rubbing, kicking out with hind limbs sometimes while nipping at the flanck, shoulders or chest, and vocalization. In extreme cases, the horse can violently lunge its body or head into a wall or other solid suject. A single episode can last to several minutes uninterrupted, and episodes can be repeated for hours over a day. In addition to bite wounds, the most common injuries are to the legs and feet from spinning and kocking. Self-mutilation is most common in Arabian, Quarterhorse and American Standardbred stallions, although it occurs in both sexes and in a variety of other breeds (e.g. A.F. Frazer, The behavior of the horse, CAB International, Oxon, 1992; L.C: Winskill et al., Appl. An Behav Sci, 1996, 48: 25-25).

Cribbing behavior is the most well known stable vice. Horses bite an object, flexes its neck, pulls back with its teeth, and swallows air. Cribbing not only damages the house surroundings, but could threaten its life. Cribbing leads to weight loss, poor performance, gaseous colic, and excessive tooth wear (e.g. N.H. Dodman et al., Am. J. Vet. Res., 1987, 48: 311-319; M. Minero et al., Proc. Measuring Behav, 1996).

Weaving behavior in horses is an odd behavior in which the horse rocks from foreleg to foreleg for long amounts of time. Weaving generally occurs in horses which are kept in stalls (e.g. A.F. Frazer, The behavior of the horse, CAB International, Oxon, 1992).

Treatment of these disorders is extremely difficult and often resistant to any treatment.

Clomipramine hydrochloride, a tricyclic antidepressant drug with serotonin (5-HT) reuptake inhibiting properties, is used in dogs (e.g. P.A. Mertens and N.H. Dodman, Kleintierpraxis, 1996, 41: 313-392; R.A. Eckstein and B.L. Hart, J. Am. An. Hosp. Assoc., 1996, 32: 225-230; R.A. Casey, Vet. Rec., 1998, 142: 587-588; A.L. Podberscek et al., Vet. Rec., 1999, 145: 365-369), cats (e.g. L.S: Saxyer et al., J. Am. Vet. Med. Assoc., 1999, 214: 71-74; K. Seksel and M.J. Lindeman, Aust. Vet. J., 1998, 76: 317-321), and birds (e.g. M.H. Grindlinger and E. Ramay, Proc. Assoc. Avian Vet., 1992). Alternatively, selective serotonin (5-HT) reuptake inhibitors (SSRIs) such as fluoxetine are used in such species including horses (e.g. K.L. Overall, Can. Pract., 1996, 21: 20-24; P.A. Mertens, ESVCE Newsletter, 1997, 3: n° 4/5; H.G. Nurnberg et al., Biol. Psychiatr., 1997, 41: 226-229; D. Wynchank and M. Berk, Depress. Anxiety, 1998, 8: 21-23; J. Romatowski, Feline Pract., 1998, 26: 14-15). Finaly, also dopamine antagonists such as haloperidol have been reported to be effective in dogs, cats, birds and horses (e.g. F. Iglauer and R.Rasim, J. Sm. An. Pract., 1993, 34: 564-566; H.G. Nurnberg et al., Biol. Psychiatr., 1997, 41: 226-229; U.A. Luescher, in: N.H. Dodman and L. Shuster (eds.): Psychopharmacology of Animal Behavior, Blackwell Science Inc., Malden, 1998, pp. 203-221).

Although serotonin 5-HT_{1A} receptor agonists are not established as a pharmacological treatment in animals, the 5-HT_{1A} receptor agonist busprione has been proposed for dogs and cats (B.L. Hart et al., J. Am. Vet. Med. Assoc., 1993, 203: 254-258; K.L. Overall, J. Am. Vet. Med. Assoc., 1994, 205: 694-696; W. Jochle, Tierärztl. Prax., 1998, 26: 410-421). But is long known that 5-HT_{1A} receptor agonists reduce stress and relieve anxiety in animals (e.g. J.E. Barrett and K.E. Vanover, Psychopharmacology, 1993, 112: 1-12; G. Griebel, Pharmac. Ther., 1995, 65: 319-395; F.G. Graeff et al., Pharmacol. Biochem. Behav., 1996, 54: 129-141) and man (e.g. J.P. Feighner et al., J. Clin. Psychiatr., 1982, 43: 103-108; A.F. Jacobson et al., Pharmacotherapy, 1985, 5: 290-296; J.J. Sramek et al., Depress. Anxiety, 1999, 9: 131-134). Moreover, 5-HT_{1A} receptor agonists have been shown be effective alone (J.P. Apter and L.A. Allen, J. Clin. Psychopharmacol., 1999, 19: 86-93) and particularly to augment the effect of SSRIs (P.J. Markovitz et al., Am. J. Psychiatr., 1990, 147: 798-800) in obsessive-compulsive disorders in humans which resemble in various aspects (e.g. trichotillomania, onychophagia, obsessive checking) self directed traumatic disorders and compulsive disorders in animals (e.g. E. Yadin et al., Pharmacol. Biochem. Behav., 1991, 40: 311-315; J.L. Rapoport et al., Arch. Gen. Psychiatr., 1992, 49: 517-521; P.S. Bordnick et al., J. Behav. Ther. Exp. Psychiatr., 1994, 25: 189-196; D.J. Stein and N.H. Dodman, Comp. Psychiatr., 1994, 35: 275-285; H.G. Nurnberg et al., Biol. Psychiatr., 1997, 41: 226-229; H. Szechtman et al., Behav. Neurosci., 1998, 112: 1475-1485, Pol. J. Pharmacol., 1999, 51: 55-61). Therefore, the combination of selective serotonin (5-HT) reuptake inhibiting properties and 5-HT_{1A} receptor agonistic properties represent an advantage over either SSRIs or 5-HT_{1A} receptor agonists alone for the treatment of self directed traumatic disorders and compulsive disorders.

Accordingly, the present invention relates to the use of compounds being combined selective serotonin (5-HT) reuptake inhibitors (SSRIs) and 5-HT_{1A} receptor agonists, in particular of 1-[4-(5-cyanoindol-3-yl)butyl]-4-(2-carbamoyl-benzofuran-5-yl)-piperazine or a physiologically acceptable salt thereof or 3-{4-[4-(4-cyano-phenyl)-piperazin-1-yl]-butyl}-1H-indole-5-carbonitrile or a physiologically acceptable salt thereof, for the manufacture of a medicament for use in veterinary medicine for the treatment of self directed traumatic disorders including acral lick dermatitis in dogs, psychogenic alopecia in cats and feather picking in birds, and/or compulsive disorders.

Accordingly, the present invention relates to the use of 1-[4-(5-cyanoindol-3-yl)butyl]-4-(2-carbamoyl-benzofuran-5-yl)-piperazine or a physiologically acceptable salt thereof, for the manufacture of a medicament for use in veterinary medicine for the treatment of disorders associated with behavioral stressors.

The present invention relates furthermore to the use of 3-{4-[4-(4-cyanophenyl)-piperazin-1-yl]-butyl}-1H-indole-5-carbonitrile or a physiologically acceptable salt thereof, for the manufacture of a medicament for use in veterinary medicine for the treatment of disorders associated with behavioral stressors.

A preferred salt of 1-[4-(5-cyanoindol-3-yl)butyl]-4-(2-carbamoyl-benzofuran-5-yl)-piperazine is 1-[4-(5-cyanoindol-3-yl)butyl]-4-(2-carbamoyl-benzofuran-5-yl)-piperazine hydrochloride. Therefore the invention relates to the use for the manufacture of a medicament for use in veterinary medicine for the treatment of disorders associated with behavioral stressors in which the pharmacologically acceptable salt of 1-[4-(5-cyanoindol-3-yl)butyl]-4-(2-carbamoyl-benzofuran-5-yl)-piperazine is 1-[4-(5-cyanoindol-3-yl)butyl]-4-(2-carbamoyl-benzofuran-5-yl)-piperazine hydrochloride.

A preferred salt of 3-{4-[4-(4-cyano-phenyl)-piperazin-1-yl]-butyl}-1H-indole-5-carbonitrile is 3-{4-[4-(4-cyano-phenyl)-piperazin-1-yl]-butyl}-1H-indole-5-carbonitrile hydrochloride.

Therefore the invention relates to the use for the manufacture of a medicament for use in veterinary medicine for the treatment of disorders associated with behavioral stressors in which the pharmacologically acceptable salt of 3-{4-[4-(4-cyano-phenyl)-piperazin-1-yl]-butyl}-1H-indole-5-carbonitrile is 3-{4-[4-(4-cyano-phenyl)-piperazin-1-yl]-butyl}-1H-indole-5-carbonitrile hydrochloride.

Additionally, the invention relates to the use of a pharmaceutical composition containing at least a compound being a combined selective serotonin (5-HT) reuptake inhibitor (SSRI) and 5-HT_{1A} receptor agonist, in particular 1-[4-(5-cyanoindol-3-yl)butyl]-4-(2-carbamoyl-benzofuran-5-yl)-piperazine or a physiologically acceptable salt thereof or 3-{4-[4-(4-cyanophenyl)-piperazin-1-yl]-butyl}-1H-indole-5-carbonitrile or a physiologically acceptable salt thereof, together with at least one solid, liquid or semiliquid excipient or adjunct for the treatment of disorders associated with behavioral stressors.

Thus the invention provides a pharmaceutical preparation for the treatment of disorders associated with behavioral stressors for use in veterinary medicine characterized in that it contains at least 1-[4-(5-cyanoindol-3-yl)butyl]-4-(2-carbamoyl-benzofuran-5-yl)-piperazine and/or one of its pharmaceutically acceptable salts and at least one auxiliary substance.

Thus the invention provides a pharmaceutical preparation for the treatment of disorders associated with behavioral stressors for use in veterinary medicine characterized in that it contains at least 3-{4-[4-(4-cyano-phenyl)-piperazin-1-yl]-butyl}-1H-indole-5-carbonitrile and/or one of its pharmaceutically acceptable salts and at least one auxiliary substance.

Suitable excipients are organic or inorganic substances which are suitable for enteral (e.g. oral), parenteral or topical adminstration and which do not react with the active compounds, for example water, vegetable oils, benzyl alcohols, alkylene glycols, polyethylene glycols, glycerol triacetate, gelatine, carbohydrates such as lactose or starch, magnesium stearate, talc, petroleum jelly. Forms which are used for oral administration are, in particular, tablets, pills, sugar-coated tablets, capsules, powders, granules, syrups, liquids or drops, forms for rectal administration are, in particular suppositories, forms for parenteral administration are, in particular, solvents, preferably oily or aqueous solutions, furthermore suspensions, emulsions or implants, and forms for topical administration are transdermal plasters, ointments, creams or powders. The active compounds may also be lyophilized and the resulting lyophilisates used for example for the preparation of injectable products. The abovementioned preparations can be in sterilized form and/or comprise auxiliaries such as glidants, preservatives, stabilizers and/or wetting agents, emulsifiers, salts for modifying the osmotic pressure, buffer substances, colourings, flavourings and/or other active ingredients, e.g. one or more vitamins.

The compounds, 1-[4-(5-cyanoindol-3-yl)butyl]-4-(2-carbamoyl-benzofuran-5-yl)-piperazine and its pharmaceutically acceptable salts or 3-{4-[4-(4-cyano-phenyl)-piperazin-1-yl]-butyl}-1H-indole-5-carbonitrile and its pharmaceutically acceptable salts, according to the invention are preferably administered in analogy to other known commercially available preparations for the treatment of disorders associated with behavioral disorders in veterinary medicine. A unit dose will generally contain from 0.1 to 300 mg, preferably between approximately 0.1 (for small birds) and 100 mg (for large dogs), in particular 0.1, 0.5, 1, 5, 10, 30, 50, 100, 200, and 300 mg. The composition may be administered once or more times a day for example 2, 3 or 4 times daily. The daily dose is preferably between approximately 1 and 10 mg/kg of body weight. However, the specific dose for each recepient animal depends not only on the species but on all sorts of factors, for example on the activity of the specific compound employed, on the age, body weight, general state of health, sex, on the diet, on the time and route of administration, on the excretion rate, pharmaceutical substance combination and severity of the particular disorder to which the therapy relates. Oral administration is preferred; the appropriate dosage can typically be mixed with the food without major difficulty, but also peroral routes of administration (*e*.*g*. intramuscular or transdermal) can be utilized.

To prove the efficacy of the compounds according to the invention, the following clinical studies are described:

### Example 1:

The aim of the study is to assess the efficacy of the compound treatment of acral lick dermatitis (ALD) in dogs in a multicenter, placebo-controlled study involving veterinarian practitioners (adopted from J.L. Rapoport et al., Arch. Gen..Psychiatr., 1992, 49: 517-521; D. Wynchank and M. Berk, Depress. Anxiety, 1998, 8: 21-23; A.L. Podberscek et al., Vet. Rec., 1999,145: 365-369).

Forty dogs with ALD are treated with the compound 2 mg/kg twice daily or , or placebo, for 8 weeks. If necessary, the dose is adjusted according to the clinical response. Owners rate both appearance of the lesion and licking behavior weekly, and veterinarians rate pre- and post-treatment lesions.

### Example 2:

The aim of the study is to assess the efficacy of the compound treatment of psychogenic alopecia in cats in a multicenter, placebo-controlled study involving veterinerian practitioners (according to K. Seksel and M.J. Lindeman, Aust. Vet. J., 1998, 76: 317-321).

Twenty-eight cats with psychogenic alopecia are treated with the compound with a starting dose of 1 mg/kg twice daily, or placebo, for 8 weeks. If necessary, the dose is adjusted according to the clinical response Owners rate both appearance and size of the lesion weekly, and veterinarians rate pre- and post-treatment.

### Example 3:

The aim of the study is to assess the efficacy of the compound treatment of feather picking in birds in a multicenter, placebo-controlled study involving veterinerian practitioners (according to P.A. Mertens, ESVCE Newsletter, 1997, n° 4/5).

Twentyfour birds with feather picking are treated with the compound 3 mg/kg threetimes daily, or placebo, for a minimum of 4 weeks and up to 8 weeks. If necessary, the dose is adjusted according to the clinical response. Owners rate both appearance and size of the feather-less lesion, and veterinarians rate pre- and post-treatment (4 weeks and 8 weeks, in case).

## Claims

1. Use of compounds being combined selective serotinin (5-HT) reuptake inhibitors (SSRIs) and 5-HT_{1A} receptor agonists for the manufacture of a medicament for the treatment of acral lick dermatitis in dogs, psychogenic alopecia in cats, feather picking in caged birds, and self-mutilation, cribbing and weaving in horses, wherein the compounds are selected from the group consisting of 1-[4-(5-cyanoindol-3-yl)butyl]-4-(2-carbamoyl-benzofuran-5-yl)-piperazine or a physiologically acceptable salt thereof or 3-{4-[4-(4-cyano-phenyl)-piperazine-1-yl]-butyl}-1H-indole-5-carbonitrile or a physiologically acceptable salt thereof.

2. Use according to claim 1 in which the physiologically acceptable salt of 1-[4-(5-cyanoindol-3-yl)butyl]-4-(2-carbamoyl-benzofuran-5-yl)-piperazine is 1-[4-(5-cyanoindol-3-yl)butyl]-2-(2-carbamoyl-benzofuran-5-yl)-piperazine hydrochloride.

3. Use according to claim 1 in which the physiologically acceptable salt of 3-{4-[4-(4-cyano-phenyl)-piperazine-1-yl]-butyl}-1H-indole-5-carbonitrile is 3-{4-[4-(4-cyanophenyl)-piperazine-1-yl]-butyl}-1H-indole-5-carbonitrile hydrochloride.

## Patentansprüche

1. Verwendung von Verbindungen, die kombinierte selektive Serotonin-(5-HT-) Reuptake-Hemmer (SSRI) und 5-HT_{1A}-Rezeptoragonisten sind, zur Herstellung eines Arzneimittels zur Behandlung von akraler Leckdermatitis bei Hunden, psychogener Alopezie bei Katzen, Federpicken bei Käfigvögeln und Selbstverstümmlung, Koppen und Weben bei Pferden, wobei die Verbindungen aus der Gruppe bestehend aus 1-[4-(5-Cyanindol-3-yl)butyl]-4-(2-carbamoylbenzofuran-5-yl)-piperazin oder einem physiologisch unbedenklichen Salz davon oder 3-{4-[4-(4-Cyanphenyl)-piperazin-1-yl]-butyl}-1H-indol-5-carbonitril oder einem physiologisch unbedenklichen Salz davon ausgewählt sind.

2. Verwendung nach Anspruch 1, wobei es sich bei dem physiologisch annehmbaren Salz von 1-[4-(5-Cyanindol-3-yl)butyl]-4-(2-carbamoyl-benzofuran-5-yl)-piperazin um 1-[4-(5-Cyanindol-3-yl)butyl]-4-(2-carbamoylbenzofuran-5-yl)-piperazin-Hydrochlorid handelt.

3. Verwendung nach Anspruch 1, wobei es sich bei dem physiologisch annehmbaren Salz von 3-{4-[4-(4-Cyanphenyl)-piperazin-1-yl]-butyl}-1 H-indol-5-carbonitril um 3-{4-[4-(4-Cyanphenyl)-piperazin-1-yl]-butyl}-1 H-indol-5-carbonitril-Hydrochlorid handelt.

## Revendications

1. Utilisation de composés qui sont des inhibiteurs sélectifs de la recapture de sérotonine (5-HT) (SSRI) et des agonistes de récepteur 5-HT_{1A} combinés, pour la fabrication d'un médicament pour le traitement de la dermatite de léchage des extrémités chez les chiens, de l'alopécie psychogène chez les chats, de l'auto-arrachage de plumes chez les oiseaux en cage et de l'auto-mutilation, du tic à l'appui et de balancement chez les chevaux, dans laquelle les composés sont choisis parmi le groupe comprenant 1-[4-(5-cyanoindol-3-yl)butyl]-4-(2-carbamoyl-benzofuranne-5-yl)-pipérazine ou son sel physiologiquement acceptable ou 3-{4-[4-(4-cyano-phényl)-pipérazine-1-yl]-butyl}-1H-indole-5-carbonitrile ou son sel physiologiquement acceptable.

2. Utilisation selon la revendication 1, dans laquelle le sel acceptable physiologiquement de 1-[4-(5-cyanoindol-3-yl)butyl]-4-(2-carbamoyl-benzofuranne-5-yl)-pipérazine est hydrochlorure de 1-[4-(5-cyanoindol-3-yl)butyl]-4-(2-carbamoyl-benzofuranne-5-yl)-pipérazine.

3. Utilisation selon la revendication 1, dans laquelle le sel acceptable physiologiquement de 3-{4-[4-(4-cyano-phényl)-pipérazine-1-yl]-butyl}-1 H-indole-5-carbonitrile est hydrochlorure de 3-{4-[4-(4-cyano-phényl)-pipérazine-1-yl]-butyl}-1H-indole-5-carbonitrile.
